# EUROPEAN PATENT APPLICATION

(11) **EP 4 775 258 A1**
(43) Date of publication of application: **15.07.2026**
(21) Application number: 25224413.2
(22) Date of filing: 17.12.2025
(51) Int. Cl.: A61N 5/10

(54) **BEAM GUIDE**

(30) Priority: 08.01.2025 EP 25150698
(71) Applicant: Vision RT Limited, London N3 2JU (GB)
(72) Inventor: WAGHORN, Benjamin James, London, N3 2JU (GB); BARRETT, Adrian Roger William, London, N3 2JU (GB)
(74) Representative: Demant

(57) **Abstract**

A monitoring system for radiotherapy comprising a processor. The monitoring being configured to obtain a radiation plan comprising one or more settings of a radiation source, obtain real-time data indicative of a surface of an object to be radiated, determine a modelled surface of the object based on the real-time data, determine a beam projection based on the radiation plan, determine a combined surface of the object by mapping the beam projection onto the modelled surface, and output the combined surface.

## Description

### TECHNICAL FIELD

The present application relates to the field of monitoring systems for radiotherapy.

### BACKGROUND

During radiotherapy it is important for the treatment beam to be delivered to the correct location, while avoiding inadvertent delivery to other areas. Historically, during patient setup the linear particle accelerator (LINAC) light field may be used to assess where the radiation beam will be delivered relative to the patient's body. The projection of the light will show how well the patient is positioned relative to the treatment field. For example, it may show some projection of light on the contralateral breast or can assist with the placement of bolus. To use the light field, the clinician is required to physically rotate the LINAC gantry to the different treatment angles, and to assess the light field shape and location on the patient.

Use of the light field is slow and labour intensive in practice, with interpretation of the light pattern on the surface sometimes proving challenging, with inter- and intra-operator variability, and a dependency on ambient room lighting. Consequently, and with increased use of image guided radiotherapy, use of the light field during setup has declined, despite the value in assessing where the treatment field will irradiate the patient. Additionally, some modern treatment systems do not include a light field, thus not allowing a pre-treatment beam alignment check.

Thus, it is an object of the present disclosure to overcome or at least mitigate the issues of the prior art.

### SUMMARY

In an aspect of the present application, a monitoring system for radiotherapy is provided. The monitoring system may comprise a processor. The monitoring system may be configured to obtain a radiation plan comprising one or more settings of a radiation source. The monitoring system may be configured to obtain real-time data indicative of a surface of an object to be radiated. The monitoring system may be configured to determine a modelled surface of the object based on the real-time data. The monitoring system may be configured to determine a beam projection based on the radiation plan. The monitoring system may be configured to determine a combined surface of the object by mapping the beam projection onto the modelled surface. The monitoring system may be configured to output the combined surface of the object.

Consequently, the disclosed monitoring system provides a virtual light field which may assist a specialist in preparing a patient to undergo radiotherapy. By providing the light field virtually, it circumvents the need for additional light sources and allows for utilizing infrastructure already in place, e.g., by utilizing 3D cameras already in place for surface guided radiotherapy. The combined surface output by the disclosed monitoring system may assist in ensuring radiation is not delivered to unintended locations by allowing pre-treatment beam alignment checks on persons undergoing treatment.

The object can be e.g., a patient or a radiotherapy phantom.

Radiotherapy may in the present context be understood as any treatment conducted with ionizing radiation, such as X-rays, protons, neutrons, electrons etc.

The monitoring system may be arranged in a treatment room comprising a radiation source for delivering radiation to an object. The monitoring system may comprise one or more cameras for receiving one or more views of an object prior to and during radiation being delivered to the object. The monitoring system may be an integral part of a system for radiotherapy. The monitoring system may be provided as a modular system which may be provided as an addon for a system for radiotherapy. In the previous and following text it is written the monitoring system is configured to perform some action, this should be interpreted broadly to not only designate the monitoring system but components of the monitoring system as well, e.g., when it is written the monitoring system is configured to do something it is equally applicable that the processor of the monitoring system is configured to perform the action, or other components of the monitoring system.

The processor in the present disclosure may be understood as a device or a system that performs operations on data according to a set of instructions. This may comprise executing arithmetic, logical, control, and input/output operations. The processor may comprise one or more processing units. The processor may be distributed over several processing units, i.e., part of the processing performed by the processor may be performed by a first processing unit and another part of the processing performed by the processor may be performed by a second processing unit.

The radiation plan may form part of a digital imaging and communication in medicine (DICOM) plan for a patient. The radiation plan may be obtained from a treatment planning system via a wired connection or a wireless connection. The radiation plan may be obtained directly from a radiation source by interfacing with radiation source via a wired connection or a wireless connection. The monitoring system may comprise a communication interface for communicating with other devices, thus allowing the radiation plan to be obtained via a communicative connection with another device. The radiation plan may be obtained by the processor of the monitoring system. The radiation plan may comprise a RTSTRUCT file. The RTSTRUCT file may comprise internal structures, such as OARs (Organs At Risk), and/or target volumes relevant to the treatment. The monitoring system may be configured to determine the combined surface by mapping the beam projection and the internal structures onto the modelled surface.

The one or more settings of a radiation source may be any setting of the radiation source which affects or otherwise modifies a radiation beam generated by the radiation source. The one or more settings of the radiation source may be linked to the generation of the radiation beam, e.g., jaw location or parameters of a multi leaf collimator. The one or more settings of the radiation source may be linked to an auxiliary device used in conjunction with the radiation source such as a couch position or couch orientation. The auxiliary device may be comprised by the radiation source. The one or more settings may be obtained via a communicative connection with the radiation source, e.g., a wired or a wireless data connection. The one or more settings may be obtained directly from the radiation source, or via another device linked to the radiation source.

The radiation source in the present disclosure may be understood as a device to be used for radiotherapy capable of emitting a beam for radiation to be used for radiotherapy. The radiation source may be a linear particle accelerator. The radiation source may be a cyclotron. The radiation source may be a synchrotron. The radiation source may further comprise auxiliary devices, such as a couch for placing an object on to undergo radiation. In the present disclosure, when stating the radiation source is capable of emitting a beam for radiation it may be understood as the radiation source being capable of emitting ionizing radiation to be used for radiotherapy.

The real-time data obtained may be any type of data indicative of a surface of an object to be radiated. The real-time data may be 3D camera data obtained by one or more 3D cameras, such as the one or more camera of the monitoring system. The real-time data may be LIDAR data obtained by one or more lasers scanning the surface of the object to be radiated. The real-time data may be obtained by the processor of the monitoring system.

The modelled surface may be understood as a virtual representation of a surface of the object to be radiated or at least part of the object to be radiated. The modelled surface is determined based on the real-time data. The modelled surface may be determined as a set of voxels. The modelled surface may be determined as a 3D surface. The modelled surface may be determined as the combination of a depth map and a texture map. The processor of the monitoring system may be configured to determine the modelled surface of the object based on the real-time data. The modelled surface may be a virtual representation of the objection to be radiated.

The beam projection may be understood as an estimated path of a radiation beam emitted by the radiation source. The beam projection may be determined based on the radiation source. The beam projection may be determined by a ray tracing algorithm based on the radiation plan. The processor of the monitoring system may be configured to determine the beam projection based on the radiation plan.

The combined surface may be a virtual representation of the modelled surface where the beam projection has been overlaid onto. The combined surface may correspond to the modelled surface where the beam projection has been overlaid onto.

Mapping of the beam projection onto the modelled surface may be achieved by using known information of a system for performing the radiotherapy. Said information may comprise geometries, such as the radiation isocenter, and the treatment plan. The planned shaped and path of the beam may be determined in 3D as a shape emanating from the radiation source. As the 3D shape intersects the modelled surface in the same coordinate system, the points of intersection define the beam projection on the modeled surface.

The combined surface may be output for further processing. The combined surface may be output to a screen for showing to people operating the radiation source and/or conducting the radiotherapy. The combined surface may provide an overview of how radiation will impact on the modelled surface. The processor of the monitoring system may be configured to output the combined surface.

When the combined surface is output, e.g. to a screen, the mapped beam projection may be displayed as an outline or a filled region projected onto the modelled surface.

When a new modelled surface is determined, a new combined surface of the object may be determined.

In an embodiment the one or more settings of the radiation source comprises one or more of the following: a gantry position, a collimator rotation, a jaw location of the radiation source, and one or more parameters of a multi leaf collimator.

In an embodiment the monitoring system is configured to:
- determine an entrance beam shape of the beam entering the object by mapping the beam projection onto the modelled surface, and output the entrance beam shape; and/or
- determine an exit beam shape of the beam exiting the object by mapping the beam projection onto the modelled surface, and output the exit beam shape.

Thus, personnel conducting the radiotherapy can get a comprehensive overview on whether radiation is only impacting onto intended areas of an object being radiated, while considering both how the beam enters and/or exits the object.

The entrance beam shape may be determined by the processor of the monitoring system. The exit beam shape may be determined by the processor of the monitoring system.

The entrance beam shape may in the present disclosure be understood as the estimated surface of the object being radiated where the radiation beam will enter the object. The entrance beam shape may be determined by overlaying the beam projection onto the modelled surface. The entrance beam shape may be viewed as a subsurface of the modelled surface.

The exit beam shape may in the present disclosure be understood as the estimated surface of the object being radiated where the radiation beam will exit the object. The exit beam shape may be determined by overlaying the beam projection onto the modelled surface. The exit beam shape may be determined by ray tracing considering interactions between the radiated object and the radiation beam, such interactions may be refraction, scattering, etc. The exit beam shape may be viewed as a subsurface of the modelled surface.

The entrance beam shape and/or the exit beam shape may be mapped onto the modelled surface to determine the combined surface.

The entrance beam shape and/or the exit beam shape may be output for further processing, e.g., to be mapped on the modelled surface or similar. The entrance beam shape and/or the exit beam shape may be output to be shown to personnel.

The beam projection determined may comprise the entrance beam shape and/or the exit beam shape.

In an embodiment the monitoring system is configured to obtain the radiation plan comprising an intended surface to be radiated or determine the intended surface to be radiated by mapping the beam projection onto a reference surface of the object to be radiated, and output the intended surface together with the combined surface. The reference surface is based on a previous capture of the surface of the object to be radiated.

Thus, personnel conducting the radiotherapy may easily compare where radiation will impact as compared to where it is supposed to impact, and in response to a misalignment the personnel may adjust correspondingly.

The intended area may be determined as part of a treatment plan for a patient, e.g., the intended area may be determined based on the placement of an area to be treated. The intended area may be determined based on prior scans, e.g., MR scan or CT imaging. The intended surface may be understood as the surface of the object to be radiated for which radiation is intended to be delivered.

The reference surface of the object to be radiated may be obtained by the one or more cameras of the monitoring system.

When the intended surface is output, e.g. to a screen, the mapped beam projection may be displayed as an outline projected onto the reference surface or a filled region projected onto the reference surface.

When the combined surface is output, e.g. to a screen, the mapped beam projection may be displayed as an outline projected onto the modelled surface or a filled region projected onto the modelled surface.

The monitoring system is configured to determine a discrepancy between the intended surface and the combined surface. The monitoring system is configured to output the discrepancy.

Consequently, even a minute discrepancy may be detected and acted upon. Furthermore, it is not reliant on personnel to notice the discrepancy, instead the discrepancy may be determined automatically by the monitoring system.

The determined discrepancy may be output for further processing. The determined discrepancy may be output as a message for personnel, e.g., as a text notification on a screen.

The discrepancy between the intended surface and the combined surface may be determined as a difference in the area delimited by the intersection between the modelled surface and the beam projection mapped onto the modelled surface, and the area delimited by the intersection between the reference surface and the beam projection mapped onto the reference surface. For example, if an entrance beam shape is determined and mapped onto the modelled surface and the reference surface, the discrepancy may be determined as the difference in area covered by the entrance beam shape mapped onto the modelled surface and the area covered by the entrance beam shape mapped onto the reference surface.

In an embodiment the monitoring system is configured to compare the discrepancy to a first threshold. The monitoring system is configured to, in response to the discrepancy exceeding the first threshold, output a notification signal.

Hence, personnel may be made aware of a discrepancy exceeding the threshold.

The first threshold may be set by personnel conducting the radiotherapy. The first threshold may be preset during fabrication and/or calibration of the monitoring system.

The notification signal may comprise a pop-up message on a screen for alerting personnel that the first threshold is being exceeded. The notification warning may comprise a warning going off to warn either by tactile stimuli, visual stimuli and/or auditory stimuli that the first threshold is being exceeded. The first threshold amount may, for example, be specified in delivery planning or treatment planning and/or be dependent on the type of treatment and/or region of interest. For example, it is conceivable that the first threshold may be an absolute value, e.g. expressed in millimeters, or a relative amount, e.g. expressed in a percentage.

In an embodiment the monitoring system is configured to compare the discrepancy to a second threshold, and, in response to the discrepancy exceeding the second threshold, outputting a stop signal.

Thus, a failsafe may be in place to stop a patient undergoing treatment to be exposed to radiation on unintended areas of their body.

The stop signal may be output to the radiation source to thereby stop or inhibit radiation treatment and/or to stop radiation treatment from being initiated. The stop signal may be understood as a control signal for controlling operation of the radiation source. The stop signal may be configured to only inhibit/stop radiation treatment while the second threshold is exceeded.

In an embodiment, the real-time data is visual data, and wherein the modelled surface comprises a three-dimensional surface of the object.

A system for obtaining visual data and generating a three-dimensional surface of the object is disclosed in WO 2004/004828 A2 a prior application by the Applicant incorporated herein by reference.

In an embodiment the monitoring system comprises a 3D camera for obtaining the real-time data.

In an embodiment the monitoring system comprises a light source, where the monitoring system is configured to project a light field using the light source based on the determined beam projection.

A possibility of the present disclosure is to use the determined beam projection by employing a projection device to project the light field corresponding to the beam projection onto the object to be radiated. Such an approach may allow personnel to see the light field-equivalent projection on the object without the need to rotate the gantry to each of the planned delivery positions, and also enable the exit light field to be displayed for beams where the gantry is below the couch, or occluded by the patient. Use of a projection device may allow for quality assurance and/or accuracy improvement by comparing the resulting light field shape with the combined surface and/or the determined beam projection.

The light field projected may be projected to provide a live representation of the determined beam projection. The light field projected may be projected to correspond with the determined beam projection.

In an embodiment the monitoring system is configured to determine one or more landmarks of the object to be radiated based on the radiation plan, and superimpose the one or more landmarks onto the modelled surface to determine the combined surface.

Thus, the personnel may be forewarned with regard to sensitive areas of a patient, e.g., organs of the patient.

The one or more landmarks may be one or more sensitive areas of the patient such as organs or other sensitive areas of the patient. The one or more landmarks may show placement of a bolus, or intended placement of medical equipment on the patient, such as one or more devices for fixating the patient. The one or more landmarks may be comprised by the radiation plan and determined based on the radiation plan by the monitoring system.

Prior to radiation treatment, a computer tomography (CT) and/or magnet resonance (MR) scan of the object may be obtained in order to prepare a treatment plan. The CT and/or MR scan may be used to define the intended area to be radiated, and mark organs at risk as landmarks. The radiation plan may obtain the landmarks and placement of the landmarks from the prior scans such as CT or MR scans.

In an embodiment the monitoring system is configured to determine a cumulative beam projection based on the radiation plan, and determine a combined surface of the object by mapping the cumulative beam projection onto the modelled surface and/or determine the intended surface by mapping the cumulative beam projection onto the reference surface.

Consequently, if the radiation source is configured to deliver radiation from different angles and positions, the radiation resulting from these may also be accounted for, so not only the initial position of the radiation source and the patient is considered.

The cumulative beam projection may be understood as a summation of the different beam projections delivered to the object during a course of radiotherapy. The cumulative beam projection may be determined as a heat map indicating areas with high intensity and areas with low intensity, e.g., if certain areas are exposed for longer than others such areas may be marked as high intensity areas. The cumulative beam projection may be mapped onto the modelled surface as a heat map. Thus, the cumulative beam projection may be an expression of the result of radiotherapy based on the current set up.

In an embodiment the monitoring system is configured to determine a dose distribution based on the radiation plan, and determine a combined surface of the object by mapping the dose distribution onto the modelled surface and/or determine the intended surface by mapping the dose distribution onto the intended surface.

According to a second aspect of the present disclosure a radiotherapy system is provided. The system comprises a radiation source configured to irradiate an object with radiation, and a monitoring system according to the first aspect of the present disclosure.

In an embodiment the radiotherapy system comprises a couch for positioning the object, where the radiotherapy system is configured to change the position of the couch relative to the radiation source based on a discrepancy, and where the monitoring system is configured to obtain the radiation plan comprising an intended surface to be radiated, or determine the intended surface to be radiated by mapping the beam projection onto a reference surface based on a previous capture of the surface of the object to be radiated, determine a discrepancy between the intended surface and the combined surface, and output the discrepancy.

Thus, automatic positioning of the couch may be achieved. The system may be configured to control the positioning of the object to minimize the discrepancy. The monitoring system may be configured to output the determined discrepancy to another device communicatively connected to the monitoring system, e.g., the radiation source.

According to a third aspect of the present disclosure, a monitoring system for radiotherapy is provided. The monitoring system may comprise a processor. The monitoring system may be configured to obtain a radiation plan comprising one or more settings of a radiation source. The monitoring system may be configured to determine a beam projection based on the radiation plan. The monitoring system may be configured to obtain an intended surface to be radiated from the radiation plan or determine the intended surface to be radiated by mapping the beam projection onto a reference surface based on a previous capture of the surface of the object to be radiated. The monitoring system may be configured to output the intended surface.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 shows a schematic block diagram of a radiotherapy system according to the present disclosure comprising a monitoring system according to the present disclosure.
FIG. 2 shows a schematic drawing of a radiotherapy system according to the present disclosure, and
FIG. 3 shows a schematic drawing of a virtual representation of an object to be radiated according to the present disclosure.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

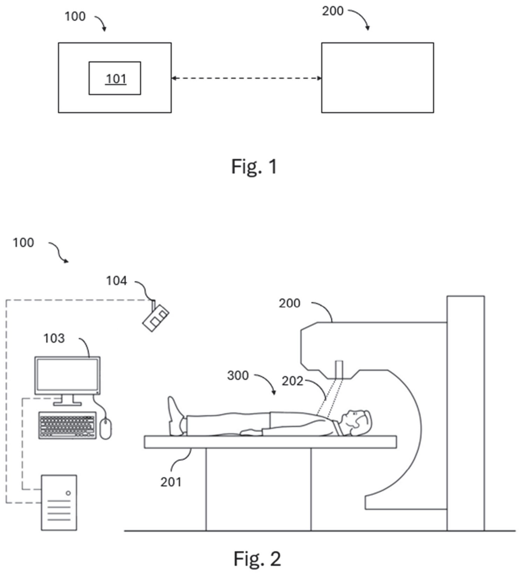

Referring initially to Fig. 1 showing a schematic block diagram of a radiotherapy system according to the present disclosure comprising a monitoring system 100 according to the present disclosure. The radiotherapy system comprises a radiation source 200. The radiation source may be machinery for performing radiotherapy. The monitoring system comprises a processor 101. The monitoring system 100 is configured to obtain a radiation plan comprising one or more settings of the radiation source 200. The monitoring system 100 is configured to obtain real-time data indicative of a surface of an object to be radiated. The monitoring system 100 is configured to determine a modelled surface of the object based on the real-time data. The monitoring system 100 is configured to determine a beam projection based on the radiation plan. The monitoring system 100 is configured to determine a combined surface of the object by mapping the beam projection onto the modelled surface. The monitoring system 100 is configured to output the combined surface of the object. The monitoring system 100 may be implemented according to the first aspect of the present disclosure. The radiotherapy system may be implemented according to the second aspect of the present disclosure.

Referring to Fig. 2 showing a schematic drawing of a radiotherapy system according to another embodiment of the present disclosure. The radiotherapy system comprises a radiation source 200 and a monitoring system 100. The radiation source 200 in the present embodiment is a linear particle accelerator configured to perform arc therapy; however, other radiation sources may be equally applicable. The radiation source 200 is configured to a radiation source configured to irradiate an object 300 with radiation. The object 300 in the present embodiment is a person 300 arranged on a couch 201. The couch 201 may be seen as part of the radiation source or at least as an auxiliary device to the radiation source 200, as the couch 201 facilitates arranging the object 300 to be irradiated in a proper position relative to the radiation source 200. The couch 201 is for positioning the object for radiotherapy. The radiotherapy system may be configured to change the position of the couch 201 relative to the radiation source 200 based on a discrepancy. The discrepancy may be determined by the monitoring system 100.

The monitoring system 100 is configured to obtain a radiation plan comprising one or more settings of the radiation source 200. The one or more settings may be a gantry position, a collimator rotation, a jaw location of the radiation source, and/or one or more parameters of a multi leaf collimator. The one or more settings may be geometric information about the radiotherapy system, such as multi leaf collimator width. The monitoring system 100 is configured to obtain real-time data indicative of a surface of an object to be radiated. The real-time data may be visual data. The visual data may be a three-dimensional surface of the object 300. The real-time data may be obtained by one or more stereoscopic cameras 104. The monitoring system 100 is configured to determine a modelled surface of the object 300 based on the real-time data. The modelled surface may be a 3D surface generated by visual data obtained by the one or more stereoscopic cameras 104. The monitoring system 100 is configured to determine a beam projection 202 based on the radiation plan. The monitoring system 100 may determine an entrance beam shape of the beam entering the object by mapping the beam projection 202 onto the surface. The monitoring system 100 may determine an exit beam shape of the beam exiting the object by mapping the beam projection 202 onto the surface. The monitoring system 100 may output the entrance beam shape and/or the exit beam shape. The monitoring system 100 is configured to determine a combined surface of the object 300 by mapping the beam projection 202 onto the modelled surface. The monitoring system 100 may be configured to obtain the radiation plan comprising an intended surface to be radiated. The monitoring system 100 may be configured to determine the intended surface to be radiated by mapping the beam projection onto a reference surface of the object to be radiated based on a previous capture of the surface of the object to be radiated. The monitoring system may be configured to output the intended surface together with the combined surface. The monitoring system 100 may be configured to determine one or more landmarks 403 of the object to be radiated based on the radiation plan. The monitoring system 100 may be configured to superimpose the one or more landmarks onto the modelled surface to determine the combined surface. The monitoring system 100 may be configured to determine a cumulative beam projection based on the radiation plan. The monitoring system 100 may be configured to determine a combined surface of the object by mapping the cumulative beam projection onto the modelled surface and/or determine the intended surface by mapping the cumulative beam projection onto the reference surface. The monitoring system 100 may be configured to determine a dose distribution based on the radiation plan. The monitoring system 100 may be configured to determine a combined surface of the object by mapping the dose distribution onto the modelled surface and/or determine the intended surface by mapping the dose distribution onto the reference surface. The monitoring system 100 is configured to output the combined surface of the object 300. The monitoring system 100 may be configured to determine a discrepancy between the intended surface and the combined surface. The monitoring system 100 may be configured to output the discrepancy. The monitoring system 100 may be configured to compare the discrepancy to a first threshold. The monitoring system 100 may be configured to, in response to the discrepancy exceeding the first threshold, outputting a notification signal. The monitoring system 100 may be configured to compare the discrepancy to a second threshold. The monitoring system 100 may be configured to, in response to the discrepancy exceeding the second threshold, outputting a stop signal.

The surfaces and signals determined by the monitoring system 100 may be output for further processing. The surfaces and signals determined by the monitoring system 100 may be output for display via a screen 103 comprised by the monitoring system 100.

The proposed monitoring system 100 may thus include calculating, and displaying on a modelled surface of the patient a beam projection during the setup of the patient and/or treatment of the patient. The beam projection may utilize information from the radiation plan to determine the beam projection. The determined beam projection may be displayed as an outline on the modelled surface. The beam projection may show virtually where the entry and exit dose would be incident onto the patient in the current position, both during patient setup, and during treatment. An intended surface to be radiated on the patient may comprise an outline of where the beam is intended to be delivered per the radiation plan, e.g., accessible from planning DICOM data. The intended surface to be radiated may be deformed to take into account patient anatomy changes, such as weight loss of swelling. The intended surface to be radiated may be used to help adjust the patient setup to align with the beam projection prior to treatment, and to evaluate accurate beam delivery during treatment. The area delimited by the intersection, in the intended surface, between the reference surface and the beam projection, may be displayed on the modelled surface in a number of ways, including as a beam outline, and/or a visual representation of the planned cumulative beam projection, again taken from the treatment plan. By projecting this area of the intended surface onto the modelled surface prior to beam delivery, the intersection of the beam projection and the reference surface and of the beam projection and the modelled surface can be visualized. Any discrepancies between the intended surface to be radiated and the combined surface can be analysed, and the patient and/or accessory setup modified, prior to the delivery of any ionizing radiation.

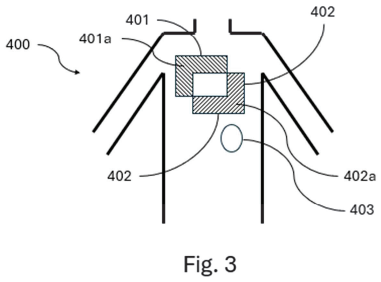

Referring to Fig. 3 showing a combined surface 400 according to an embodiment of the present disclosure. The combined surface 400 shown is an example of how the combined surface 400 may be output for viewing to personnel conducting the radiotherapy. The combined surface 400 is a modelled surface of a torso of a patient on which several other surfaces have been mapped. The combined surface 400 comprises a beam projection 401 mapped onto the modelled surface. The combined surface 400 is output with an intended surface to be irradiated comprising an area 402 delimited by the intersection between the reference surface and the beam projection mapped onto the reference surface. In the shown embodiment, a discrepancy 401a, 402a is present between the combined surface 400 and the intended surface to be irradiated. The discrepancy 401a, 402a is visualized as hatched areas of the combined surface 400 and the intended surface to be irradiated 402. The combined surface 400 comprises a landmark 403 mapped onto the modelled surface.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. A monitoring system for radiotherapy comprising a processor, and being configured to:
obtain a radiation plan comprising one or more settings of a radiation source,
obtain real-time data indicative of a surface of an object to be radiated,
determine a modelled surface of the object based on the real-time data,
determine a beam projection based on the radiation plan,
determine a combined surface of the object by mapping the beam projection onto the modelled surface, and
output the combined surface.

2. A monitoring system according to claim 1, wherein the one or more settings of the radiation source comprises one or more of the following: a gantry position, a collimator rotation, a jaw location of the radiation source, and one or more parameters of a multi leaf collimator.

3. A monitoring system according to claim 1 or 2, wherein the monitoring system is configured to:
determine an entrance beam shape of the beam entering the object by mapping the beam projection onto the surface, and output the entrance beam shape; and/or
determine an exit beam shape of the beam exiting the object by mapping the beam projection onto the surface, and output the exit beam shape.

4. A monitoring system according to any of the preceding claims, wherein the monitoring system is configured to:
obtain the radiation plan comprising an intended surface to be radiated, or
determine the intended surface to be radiated by mapping the beam projection onto a reference surface of the object to be radiated based on a previous capture of the surface of the object to be radiated, and
output the intended surface together with the combined surface.

5. A monitoring system according to claim 4, wherein the monitoring system is configured to:
determine a discrepancy between the intended surface and the combined surface, and
output the discrepancy.

6. A monitoring system according to claim 5, wherein the monitoring system is configured to:
compare the discrepancy to a first threshold,
in response to the discrepancy exceeding the first threshold, outputting a notification signal.

7. A monitoring system according to claim 5 or 6, wherein the monitoring system is configured to:
compare the discrepancy to a second threshold,
in response to the discrepancy exceeding the second threshold, outputting a stop signal.

8. A monitoring system according to any of the preceding claims, wherein the real-time data is visual data, and wherein the modelled surface comprises a three-dimensional surface of the object.

9. A monitoring system according to any of the preceding claims, wherein the monitoring system comprises a light source, wherein the monitoring system is configured to:
project a light field using the light source, based on the determined beam projection.

10. A monitoring system according to any of the preceding claims, wherein the monitoring system is configured to:
determine one or more landmarks of the object to be radiated based on the radiation plan, and
superimpose the one or more landmarks onto the modelled surface to determine the combined surface.

11. A monitoring system according to any of claims 4 to 7, wherein the monitoring system is configured to:
determine a cumulative beam projection based on the radiation plan,
determine the combined surface of the object by mapping the cumulative beam projection onto the modelled surface and/or determine the intended surface by mapping the cumulative beam projection onto the reference surface.

12. A monitoring system according to any of claims 4 to 7, wherein the monitoring system is configured to:
determine a dose distribution based on the radiation plan,
determine the combined surface of the object by mapping the dose distribution onto the modelled surface and/or determine the intended surface by mapping the dose distribution onto the intended surface.

13. A radiotherapy system comprising:
a radiation source configured to irradiate an object with radiation; and
a monitoring system comprising a processor configured to:
obtain a radiation plan comprising one or more settings of a radiation source,
obtain real-time data indicative of a surface of an object to be radiated,
determine a modelled surface of the object based on the real-time data,
determine a beam projection based on the radiation plan,
determine a combined surface of the object by mapping the beam projection onto the modelled surface, and
output the combined surface of the object.

14. A radiotherapy system according to claim 13 comprising:
a couch for positioning the object, wherein the radiotherapy system is configured to change the position of the couch relative to the radiation source based on a discrepancy, wherein the monitoring system is configured to:
obtain the radiation plan comprising an intended surface to be radiated, or
determine the intended surface to be radiated by mapping the beam projection onto a reference surface based on a previous capture of the surface of the object to be radiated, and
determine a discrepancy between the intended surface and the combined surface, and
output the discrepancy.

15. A monitoring system for radiotherapy comprising a processor, and being configured to:
obtain a radiation plan comprising one or more settings of a radiation source,
determine a beam projection based on the radiation plan,
obtain an intended surface to be radiated from the radiation plan or determine the intended surface to be radiated by mapping the beam projection onto a reference surface based on a previous capture of the surface of the object to be radiated,
output the intended surface.
